# EUROPÄISCHE PATENTSCHRIFT

(11) **EP 1 447 700 B1**
(45) Veröffentlichungstag und Bekanntmachung des Hinweises auf die Patenterteilung: **16.04.2008**
(21) Anmeldenummer: 04100467.2
(22) Anmeldetag: 09.02.2004
(51) Int. Cl.: G02B 21/24, G02B 21/00

(54) **Operationsmikroskop mit Stativ und Schwingungsausgleichseinrichtung**
Surgical microscope with stand and vibration compensating system
Microscope chirurgical avec support et dispositif à compensation de vibrations

(30) Priorität: 15.02.2003 DE 10306440
(43) Veröffentlichungstag der Anmeldung: 18.08.2004
(73) Patentinhaber: Leica Microsystems (Schweiz) AG, 9435 Heerbrugg (CH)
(72) Erfinder: Sander, Ulrich, 9445 Rebstein (CH)
(74) Vertreter: Rosenich, Paul

(56) Entgegenhaltungen:
- DE-A- 4 342 538
- US-A1- 2001 024 320
- US-A1- 2003 016 301
- MELCHER J ET AL: "MODERN ADAPTIVE REAL-TIME CONTROLLERS FOR ACTIVELY REACTING FLEXIBLE STRUCTURES" JOURNAL OF INTELLIGENT MATERIAL SYSTEMS AND STRUCTURES, TECHNOMIC PUBL., LANCASTER, PA, US, Bd. 2, Juli 1991 (1991-07), Seiten 328-346, XP008039968 ISSN: 1045-389X
- E J BREITBACH ET AL.: "Adaptive Structures - Concepts and Prospects" 1993, INSTITUTE OF AEROELASTICITY, INSTITUTE OF STRUCTURAL MECHANICS, DLR , GERMANY , XP008031297 * das ganze Dokument *

## Beschreibung

Die Erfindung betrifft ein Mikroskop mit Stativ, insbesondere ein Operationsmikroskop.

Operationsmikroskope werden von Stativen getragen, die entweder auf dem Fußboden stehen oder an Wand oder Decke montiert sind. Durch die Benutzung des Mikroskops mit seinen Funktionen wieXY-Ver stellung, Einstellen des Zooms, Lösen der Bremsen In den Gelenkarmen oder durch externe Schwingungen, die z.B. durch Erschütterungen vorbeifahrender LKWs durch unzureichend schwingungsisolierte Klimaanlagen verursacht werden, kann das Mikroskop in Vibrationen geraten, was eine sichtbare Unruhe des Mikroskopbildes zur Folge hat.

Um eine sichtbare Unruhe zu vermeiden, gibt es Lösungen, die bei Foto-Objektiven und Feldstechern eingesetzt werden, wie beispielsweise in US-B1-6 226 458, US-B1-6 191 813, US-A-5 940 630, US-A-5 561 498, US-A-5 335 032, US-A-5 245 378, US-A-5 243 462, US-A-5 182 671, US-A-5 126 561, US-A-5 107 293, US A-5 101 230, US-A-4 970 540 und US-A-3 942 862 beziehungsweise in der "Zeiss Information" 4, (1995) Nr. 6, Seite 24, veröffentlicht wurde. Es wird bei diesen bekannten Lösungen die störende Schwingung entweder elektronisch oder mechanisch erfasst und danach ein optisches Bauelement - beispielsweise eine Linse oder ein Prisma - schwingungsausgleichend bewegt bzw. verstellt. Diese Bewegung bzw. Verstellung erfolgt geregelt, und zwar entweder mittels eines motorischen Antriebs oder durch mechanische Hebelkräfte. In US-A-5 731 896, US-A-5 786 936, DE-A-43 42 717 und DE-A-43 42 538 werden auch vergleichbare Systeme für Operationsmikroskope angegeben. Auch hier werden Schwingungen des Systems gemessen, um damit ein optisches Element, in diesem Falle das Hauptobjektiv, gegenzusteuern.

Die folgende Beschreibung, insbesondere die der Nachteile, beschränkt sich auf Operationsmikroskope, obwohl die Erfindung auch bei anderen Geräten erfolgreich eingesetzt werden kann. In der US-A-5 731 896, US-A-5 786 936, DE-A-43 42 717 und DE-A-43 42 538 wird eine Lösung für Operationsmikroskope angegeben, wo die Schwingungen des Systems gemessen werden. Motore, die das Hauptobjektiv relativ zum Mikroskop in der XY-Ebene bewegen können, gleichen diese Schwingung aus, so dass das Objektbild in relativer Ruhe im Okular erscheint.

Ein wichtiger Nachteil entsteht bei dieser bekannten Lösung dadurch, dass das Objektiv in seiner Schwingung nur in der XY-Ebene ausgeglichen wird. In Z-Richtung schwingt es ungedämpft weiter. Somit gehen Schwingungen, die von der Tiefenschärfe des Objektivs nicht mehr ausgeglichen werden können, stark zu Lasten der Kontrastschärfe des Objektbildes.

Ein weiterer Nachteil ist auch, dass das gesamte Mikroskop immer noch relativ zum beobachtenden Chirurgen schwingen kann und dieser unter Umständen durch eine zu große Schwingung die Austrittspupille des Mikroskops verlieren kann bzw. ein Bild mit Vignettierung zu sehen bekommt.

Um letzteren Nachteil zu vermeiden, bietet es sich an, das gesamte optische System gegen Schwingungen auszugleichen. Solche Lösungen wurden z.B. in US-A-5 731 896, US-A-5 786 936, DE-A-43 42 717 und DE-A-43 42 538 vorgeschlagen.

Hierbei bewegen Motore das gesamte Mikroskop in der XY-Ebene, um Schwingungen auszuglelchen, so dass das Objektbild in relativer Ruhe Im Okular erscheint. Jedoch wird auch hier kein Ausgleich in Z-Richtung vorgenommen. Die Motoren müssen dabei nachteiligerweise größere Massen bewegen.

Der Erfindung liegt somit die Aufgabe zugrunde, eine leichte und kompakte Schwingungsausgleichseinrichtung für das gesamte optische System zu finden, welche die Schwingungen über die X- und Y-Achse hinaus auch In derZ-Achse und somit dreidimensional ausgleicht. Diese Aufgabe wird auf folgendem Weg gelöst:

Als Schwingungsausgleichseinrichtung wird ein ARES (Actively Reacting Flexible Structure)-Bauelement in einem oder anstelle eines Teils des Stativs angeordnet. Solche ARES-Bauelemente sind in dem Artikel von E. J. Breitbach et al: "Adaptive Structures- Concepts and Prospects" des Institute of Aeroelasticity und des Institute of Structural Mechanics des German Aerospace Research Establishment (DLR), Seiten 3-8, (1993), beschrieben. Ein ARES-Bauelement ist ein selbstregulierendes Bauelement, das aufgrund der Messung von Schwingungen integrierte Antriebselemente so ansteuert, dass diese der Schwingung in Ist-Zeit so entgegenwirken, dass die Schwingung nicht zu einer Lageveränderung der äußeren Konturen bzw. der entscheidenden Schnittstellen an dem Bauteil führen. Beschleunigungssensoren im ARES-Bauelement selbst messen diese Schwingungen und gleichen diese direkt über eine Variation des ARES-Bauelements in XYZ-Richtung aus, so dass sich das gesamte Mikroskop erfindungsgemäß relativ zum Beobachter, bzw. relativ zum Raum, in allen drei Raumrichtungen in Ruhe befindet.

Ein weiterer Vorteil der Erfindung besteht in einer Reduktion der Anzahl der Bauteile, sofern diese ARES-Bauelemente den Tragarm direkt ersetzen, also gleichzeitig als tragendes und nicht nur als schwingungsausgleichendes Element dienen. Damit werden voluminöse Motore großer Masse mit Sensoren im Bereich des Mikroskops oder zusätzliche optische Ausgleichselemente sowie eine sich daraus ergebende Sichtbehinderung vermieden. Im Sinne der Offenbarung dieser Anmeldung ist auf den Inhalt des erwähnten Artikels von Breitbach et al verwiesen.

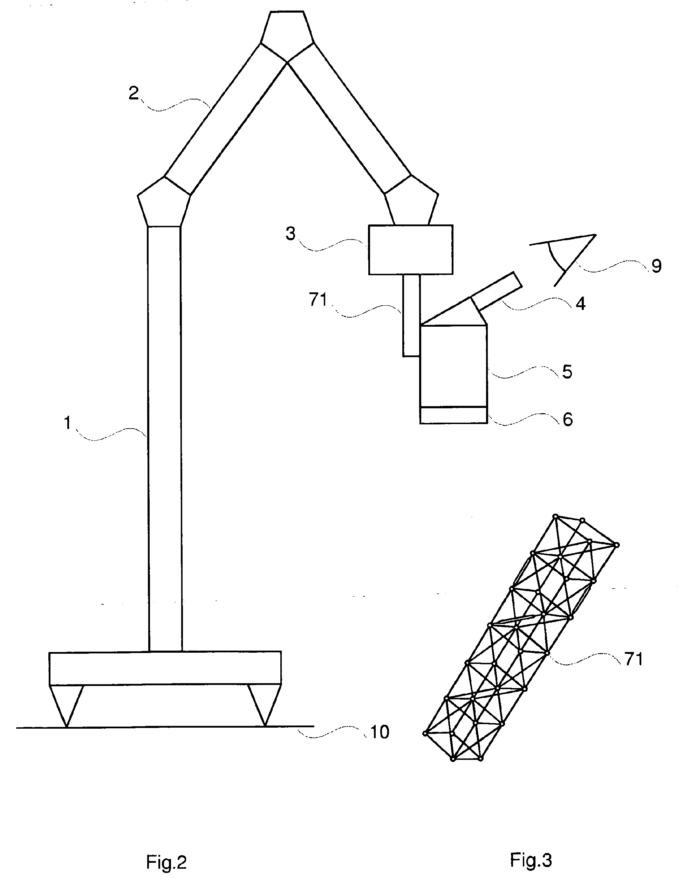

Weitere Ausbildungen der Erfindung sind in den Figuren 2 und 3, sowie in den abhängigen Patentansprüchen angegeben.

Das Stativ bedient sich eines ARES-Bauelements an einem potentiellen Auftrittsort von Schwingungsbäuchen oder mehrerer ARES-Bauelemente an verschiedenen Stellen zum Schwingungsausgleich.

Ein ARES-Bauelement kann als selbsttragendes Element anstelle eines tragenden Teils zur Schwingungsentkopplung des Mikroskops vom übrigen Stativ, d.h. zwischen Stativ und Mikroskop, angeordnet sein. Das ARES-Bauelement kann nicht nur zum Schwingungsausgleich, sondern auch schwingungsfrei zur Lagestabilisierung angesteuert werden. Ein ARES-Bauelement kann piezoelektrisch versorgt werden und somit gegebenenfalls unabhängig von üblichen Stromkreisen funktionieren. Dabei werden Piezokristalle durch die Formänderung zur Spannungserzeugung angeregt, wobei diese Spannung verwendet wird, um andere Piezoaktuatoren zur Gegensteuerung anzuregen. Diese Ausführungen können sich auch auf Stereo-Operationsmikroskope beziehen.

Anhand von schematischen Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche Bauteile an.

Es zeigen dabei:
- Fig. 1:: ein bekanntes Operationsmikroskop in Seitenansicht;
- Fig. 2:: eine Seitenansicht eines erfindungsgemäßen Operationsmikroskops mit Stativ und
- Fig. 3:: ein ARES-Bauelement in perspektivischer Darstellung.

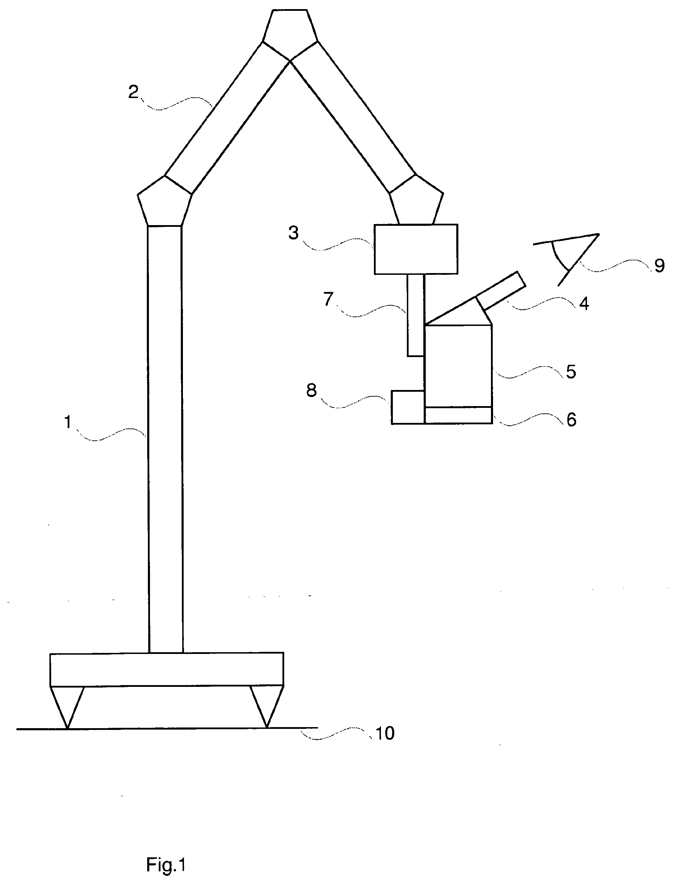

In Fig. 1 ist ein auf dem Fußboden 10 stehendes Operationsmikroskop 5 mit Stativ 1 gemäß dem Stand der Technik dargestellt. Das Stativ 1 weist einen beweglichen Scherenarm 2 zur freien Positionierung im Raum auf, an dessen freiem Ende eine XY-Verstellung 3 zur gezielten Präzisionsausrichtung durch den Beobachter angeordnet ist. Hier ist mittels eines Tragarms 7 das Mikroskop 5, inklusive Hauptobjektiv 6 und Tubus mit Okular 4, für die Beobachteraugen 9 angebracht.

Ein Motor mit Sensoren 8 zum Schwingungsausgleich befindet sich unmittelbar im Bereich des Hauptobjektivs 6, wo er als Stellglied auf dieses einwirkt. Der Motor mit Sensoren 8 kann gemäß dem Stand der Technik auch zwischen Scherenarm 2 und Tragarm 7 oder zwischen Tragarm 7 und Mikroskop 5 angebracht sein, wie in Fig.1 nicht dargestellt.

In Fig. 2 ist ein auf dem Fußboden 10 stehendes erfindungsgemäßes Operationsmikroskop 5 mit Stativ 1 dargestellt. Das Stativ 1 weist einen beweglichen Scherenarm 2 zur freien Positionierung im Raum auf, an dessen Ende eine XY-Verstellung 3 zur gezielten Präzisionsausrichtung durch den Beobachter angeordnet ist. Hier ist jedoch anstelle eines Tragarmes 7 gemäß Fig. 1 ein ARES-Bauelement 71 angeordnet, welches das Mikroskop 5 inklusive Hauptobjektiv 6 und Tubus mit Okular 4 trägt.

In Fig. 3 ist ein Beispiel eines ARES-Bauelements 71 dargestellt, das eine Raumgitterstruktur aufweist und beim erfindungsgemäßen Mikroskop den Tragarm 7 sowie die Funktion des Motors mit Sensoren 8 des in Fig. 1 beschriebenen Mikroskops ersetzt und diese somit erübrigt.

### Bezugszeichenliste

- 1 -: Stativ
- 2 -: Scherenarm (tragendes Teil)
- 3 -: XY-Verstellung
- 4 -: Tubus mit Okular
- 5 -: (Operations-)Mikroskop
- 6 -: Hauptobjektiv
- 7 -: Tragarm (tragendes Teil)
- 8 -: Motor mit Sensoren
- 9 -: Beobachterauge(n)
- 10 -: Fußboden
- 71: ARES-Bauelement

## Patentansprüche

1. Operationsmikroskop (5) an einem Stativ (1) mit wenigstens einem Tragarm aus wenigstens einem tragenden Teil (2,7) und mit einer Schwingungsausgleichseinrichtung,
**dadurch gekennzeichnet, dass**
der wenigstens eine Teil (2,7) wenigstens zum Teil als ARES-Bauelement (71) ausgebildet ist, wobei das ARES-Bauelement an dem Teil des wenigstens einen Teils ausgebildet ist, an dem potentiell Schwingungsbäuche auftreten und wobei das ARES-Bauelement ein selbstregullerendes Bauelement ist, das aufgrund der Messung von Schwingungen integrierte Antriebselemente so ansteuert, dass diese der Schwingung in Ist-Zelt so entgegenwirken, dass die Schwingung nicht zu einer Lageveränderung der äußeren Konturen bzw. der entscheidenden Schnittstellen an dem Bauteil führen, so dass sich das gesamte Mikroskop relativ zum Beobachter, bzw. relativ zum Raum, In allen drei Raumrichtungen in Ruhe befindet und dass das ARES-Bauelement als eine Raumgitterstruktur aufgebaut ist.

2. Operationsmikroskop (5) nach Anspruch 1,
**dadurch gekennzeichnet, dass**
mindestens zwei verschiedene Teile (2,7) des Stativs wenigstens zum Teil als ARES-Bauelemente (71) ausgebildet sind, wobei die ARES-Bauelemente an dem Teil der verschiedenen Teile ausgebildet sind, an dem potentiell Schwingungsbäuche auftreten.

3. Operationsmikroskop (5) nach einem der vorhergehenden Ansprüche,
**dadurch gekennzeichnet, dass**
das bzw. die ARES-Bauelement(e) eine piezoelektrische Energieversorgung aufweist (aufweisen).

## Claims

1. Surgical microscope (5) on a stand (1) with at least one carrying arm made of at least one carrying part (2, 7) and with an oscillation compensation device, **characterized in that** the at least one part (2, 7) is at least partially in the form of an ARES component (71), wherein the ARES component is formed an that part of the at least one part where oscillation antinodes potentially occur and wherein the ARES component is a self-regulating component which drives integrated drive elements on account of the measurement of oscillations such that the drive elements counteract the oscillation in real time in such a manner that the oscillation does not result in a changed position of the outer contours or of the decisive points of intersection on the component, with the result that the entire microscope is at rest relative to the observer, or relative to the space, in all three spatial directions, and **in that** the ARES component is structured as a spatial lattice structure

2. Surgical microscope (5) according to Claim 1, **characterized in that** at least two different parts (2, 7) of the stand are at least partially in the form of ARES components (71), wherein the ARES components are formed on that part of the different parts where oscillation antinodes potentially occur.

3. Surgical microscope (5) according to one of the preceding claims, **characterized in that** the ARES component(s) has/have a piezoelectric power supply.

## Revendications

1. Microscope chirurgical (5) disposé sur un statif (1) qui présente au moins un bras de support constitué d'au moins une partie portante (2, 7) et un dispositif de compensation des oscillations,
**caractérisé en ce que**
la ou les parties (2, 7) sont formées au moins en partie comme composant ARES (71), le composant ARES étant formé sur la partie de la ou des parties sur laquelle peuvent survenir des bombements oscillants, le composant ARES étant un composant auto-régulé qui, en fonction des oscillations mesurées, commande des éléments d'entraînement intégrés, **en ce que** ces derniers s'opposent en temps réel aux oscillations de telle sorte que les oscillations n'entraînent pas une modification de la position du contour extérieur ou des interfaces essentielles du composant de telle sorte que l'ensemble du microscope soit au repos par rapport à l'observateur et par rapport à l'espace dans les trois directions de l'espace, et **en ce que** le composant ARES est formé d'une structure en treillis spatial.

2. Microscope chirurgical (5) selon la revendication 1, **caractérisé en ce qu'**au moins deux parties (2, 7) différentes du statif sont configurées au moins en partie comme composants ARES (71), les composants ARES étant formés sur la partie des différentes parties sur laquelle des bombements oscillants peuvent survenir.

3. Microscope chirurgical (5) selon l'une des revendications précédentes, **caractérisé en ce que** le ou les composants ARES sont alimentés piézoélectriquement en énergie.
